# EUROPEAN PATENT APPLICATION

(11) **EP 1 319 361 A1**
(43) Date of publication of application: **18.06.2003**
(21) Application number: 01610127.1
(22) Date of filing: 17.12.2001
(51) Int. Cl.: A61B 3/032

(54) **Device for vision testing**

(71) Applicant: Maico Diagnostic GmbH, 13629 Berlin (DE)
(72) Inventor: Köpke, Wolfgang, 13629 Berlin (DE)
(74) Representative: Christensen, Mikael T.

(57) **Abstract**

The invention relates to an equipment and a method for performing vision screening. The equipment comprises a headworn module adapted to be maintained in position on the head of a test subject, where at least one display is located in the module, where the display is controllable by a control unit. The invention further relates to a similar stationary equipment as well as a method for performing vision screening

## Description

### BACKGROUND OF THE INVENTION

Vision testing or screening is today a procedure that is carried out by occupational health specialists e.g. opticians or eye specialists. For the purpose of testing or screening the vision a device is normally used, which has the capability of displaying various pictures and geometric constellations for the user. The users vision is hereby normally shielded from the environment.

During the test procedure a test specialist instructs a test subject and informs the test subject about the procedure. The test subject indicates to a test specialist, what is actually seen on the display and the test specialist will based on this information make the evaluation of the vision of the test subject.

The vision test or screening devices known today are all relatively large static constructions requiring that the test subject takes an uncomfortable position and requiring that the test subject remains in this position throughout the test procedure.

It is therefore an object of the invention to provide equipment, which may deal with this problem and hence help in achieving better results while performing vision screening or testing.

### SUMMARY OF THE INVENTION

This object is achieved by the device described in claim 1.

By this construction the test subject can achieve a more comfortable position during the test procedure and may even move his/her head or may move around.

In a preferred embodiment the device where eye movement detection means are provided to detect the response expressed through the test subjects eyes. In this embodiment the advantage of eliminating the else required subjective response of the test subject can be eliminated to at least some extent.

In further preferred embodiments means for playback of test procedure instruction to the test subject are provided, e.g. based on the detected response. This feature allows for an automated procedure reducing or eliminating the need for a test specialist being present during the entire test procedure.

The invention further relates to a method for performing a vision screening.

The method comprises the display of desired picture to a test subject and successively performing a test of the response of the test subject by detecting the eye response to the displayed picture. By this method a more objective test result may be obtained, as this no longer depends on the test subjects active response.

In a preferred embodiment instructions are given during the vision screening to the test subject based on the eye response of the test subject. This embodiment of the method can eliminate or at least reduce the need of the presence of a test specialist during the test. This will enable the test of several subjects simultaneously with a widely reduced need of test specialist presence.

In a further preferred embodiment the instructions are chosen from a selection of stored instructions based on the eye response of the test subject.

The invention finally relates to an equipment, which need not be portable but still will be able to provide improvements in the test results as a more objective test result may be obtained, due to the fact that this no longer depends entirely on the test subjects active response.

The invention will be described more detailed in the following description of a preferred embodiment with reference to the drawings.

### BRIEF DESCRIPTION OF THE DRAWINGS

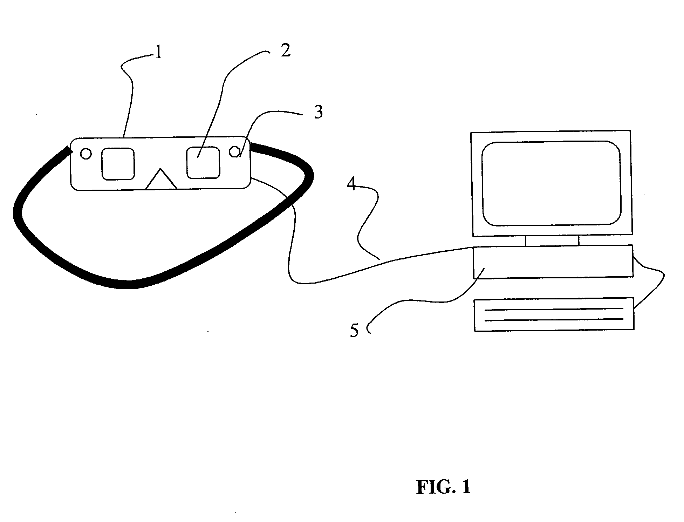

FIG. 1 shows a schematic circuit forming part of the device according to the invention.

### DETAILED DESCRIPTION OF THE INVENTION

From FIG. 1 the equipment appears schematically.

The equipment comprises a headworn module in the form of a mask adapted to be fastened and maintained in position in relation to the head of a test subject. In the mask two TFT displays are located in a position in front of the test subject's eyes. In this embodiment TFT screens are mentioned as the preferred option, however other types of displays or screens may be adapted. The displays are controllable by control means, which may comprise a computer that is operatively connected to the and to the displays. The computer may have in its memory a selection of standard tests, including pictures and geometrical figures that can be transmitted to the displays and shown to the test subject. This may follow standard test procedures and the test subject will compared to previously known test equipment have the possibility of taking a more comfortable position and may even be able to change position during the test procedure. The test subject hence experiences a more comfortable test procedure and the test results may improve due to a reduced amount of stress applied to the test subject.

The mask further comprises two miniature video cameras, one located at each of the test subject's eyes. The purpose of these cameras is to record the eye response to the picture displayed on the TFT display. The recorded response is transmitted to a computer connected to the cameras and processed in this computer according to specified criteria by use of the software adapted for this purpose. The software generates a conclusion on the recorded response and stores this as part of the test result.

Based on the conclusion the computer with its preprogrammed procedures generates an output in the form of instruction to the test subject. These instructions can either be repeating instructions or instructions for a successive part of a predetermined test procedure. This allows for reducing the necessity of the presence of a test specialist during the entire procedure. This also enables the simultaneous test of several test subjects by a single test specialist.

The same computer may control several masks or stationary devices

The equipment may be established as a stationary device, which will provide the improvements as to the increased objectivity of the test, where this however does not allow the same mobility as in connection with the headworn device.

## Claims

1. An equipment for use in vision screening, the equipment comprising a headworn module adapted to be maintained in position on the head of a test subject, where at least one display is located in the module, where the display is controllable by a control unit.

2. An equipment according to claim 1, where response detection means are provided to detect a response expressed through the test subjects eyes.

3. An equipment according to claim 1 or 2, where means for playback of test procedure instruction to the test subject are provided, e.g. in the headworn module.

4. An equipment according to claim 3, where control means are provided for controlling the playback of test procedure instructions and where the control means are responsive to the output of the response detection means.

5. A method for performing a vision screening comprising the display of desired picture to a test subject and successively performing a test of the response of the test subject by detecting the response to the displayed picture expressed through the test subjects eye.

6. A method according to claim 5, where during the vision screening instructions are given to the test subject based on the eye response of the test subject.

7. A method according to claim 5 or 6, where the instructions are chosen from a selection of stored instructions based on the eye response of the test subject.

8. A method according to claim 5, 6 or 7, where during the vision screening the test procedure changes adaptive to the eye response of the test subject.

9. An equipment for use in vision screening, the equipment comprising a module having at least one display located in the module, where the display is controllable by a control unit and response detection means are provided to detect a response expressed through the test subjects eyes.

10. An equipment according to claim 9, where means for playback of test procedure instruction to the test subject are provided.

11. An equipment according to claim 9 or 10, where control means are provided for controlling the playback of test procedure instructions and where the control means are responsive to the output of the response detection means.
